# EUROPEAN PATENT APPLICATION

(11) **EP 2 193 771 A1**
(43) Date of publication of application: **09.06.2010**
(21) Application number: 08778071.4
(22) Date of filing: 11.07.2008
(51) Int. Cl.: A61F 13/15, A61F 13/49, A61F 13/494, A61F 13/511

(54) **ABSORPTIVE ARTICLE**

(30) Priority: 28.09.2007 JP 2007255991
(71) Applicant: Unicharm Corporation, Ehime 799-0111 (JP)
(72) Inventor: NAKAJIMA, Kaiyo, Kanonji-shi Kagawa 769-1602 (JP); MINATO, Hironao, Kanonji-shi Kagawa 769-1602 (JP)
(74) Representative: Stark, Gordon Drummond
(86) International application number: PCT/JP2008/062555
(87) International publication number: WO 2009/041144

(57) **Abstract**

The present invention aims to provide an improved absorbent article which overcomes the problem that the upward spacing of the barrier sheet from the liquid-absorbent structure might prevent body waste from being smoothly guided through the passage openings into the space adapted to receive body waste. A liquid-absorbent chassis 2 is provided on the inner surface 100 facing the wearer' s skin, with a barrier sheet 8 which is, in turn, formed with front and rear passage openings 27, 28. A sheet region of the barrier sheet 8 defined between barrier sheet elastic members 34L, 34R and side edges 40, 41 is provided with tucks 42, 43 serving as means to prevent the sheet region from sagging toward the front and rear passage openings 27, 28. These tucks 42, 43 are formed by folding back the sheet in the sheet region onto itself. These tucks 42, 43 extend in a longitudinal direction Y in regions of opposite lateral portions 44, 45 including convex segments of the barrier sheet elastic members 34L, 34R extending therein.

## Description

### TECHNICAL FIELD

The present invention relates to an absorbent article and particularly to an absorbent article such as disposable diaper, toilet training pants or incontinent briefs.

### RELATED ART

Disposable diapers which have a specific construction adapted to protect the diaper wearer's skin from contamination with body waste are well known, for example, from PCT International Application Publication No. 1997-510385 (JP 1997-510385W).
PATENT DOCUMENT: PCT International Application Publication No. 1997-510385 (JP 1997-510385W)

### DISCLOSURE OF THE INVENTION

### PROBLEM TO BE SOLVED BY THE INVENTION

JP 1997-510385W discloses a pant-type disposable diaper. This diaper comprises a pant-shaped cover sheet and a liquid-absorbent structure provided on the inner side of the cover-sheet, wherein the liquid-absorbent structure is provided thereon with a pair of sheet-like flaps spaced from each other in a transverse direction. The paired flaps are joined to each other at a joining portion which cooperates with the respective flaps to define a front passage opening and a rear passage opening. The flaps are provided along these front and rear passage openings with elastic members which are attached under tension to the respective flaps so as to extend in a longitudinal direction. Under contraction of these elastic members, the sheet regions defined between the elastic members and the opposite side edges of the flaps are spaced upward together with the joining portion from the liquid-absorbent structure so that a pocket to receive urine is formed under the front passage opening and a pocket to receive feces is formed under the rear passage opening.

Upon putting the diaper on the wearer's body, the crotch region bows and the sheet regions of the respective flaps rise up may more or less sag. The sheet regions slacking in this manner may, under a load such as the body weight of the wearer, further sag towards the passage openings and eventually occlude or at least reduce the areas of the respective passage openings. As a consequence, there is the possibility that the smooth passage of body waste may be obstructed during its passage through the passage openings into the pocket.

In view of the problem as has been described above, the present invention provides an improved absorbent article which is of a construction which allows the wearer to be free from anxiety that the barrier sheet, when spaced upward from the liquid-absorbent structure, might prevent body waste from being smoothly guided through the passage openings into the space adapted to receive body waste.

### MEASURE TO SOLVE THE PROBLEM

According to the present invention, there is provided absorbent article basically comprising a liquid-absorbent chassis having a longitudinal direction, a transverse direction, a side facing the article wearer's body, a side facing the wearer's garment, a front waist region, a rear waist region, a crotch region extending between the front and rear waist region and a liquid-absorbent structure provided in the crotch region, and a barrier sheet provided on the side facing the article wearer's body of the liquid-absorbent chassis which is adapted to be spaced from the liquid-absorbent chassis, wherein the barrier sheet includes barrier sheet elastic members attached under tension thereto which extend in the longitudinal direction, and a space which is formed between the barrier sheet and the liquid-absorbent structure, as the liquid-absorbent structure is bowed in the longitudinal direction.

The improvement according to the present invention is **characterized in that** the barrier sheet comprises a pair of lateral portions opposed to and spaced from each other in the transverse direction, a middle portion connecting the lateral portions to each other, front and rear passage openings defined by the lateral portions and the middle portion, through which body waste can be guided into the space and lateral portions which are attached at front and rear end portions to the front and rear waist regions, the barrier sheet elastic members are attached to the lateral portions and extend along the passage openings, and the barrier sheet is provided with means adapted to prevent a sheet region of the barrier sheet extending outside the barrier sheet elastic members as viewed in the transverse direction from sagging toward the passage openings.

According to one preferred embodiment, the means comprise tucks formed by at least partially folding back the sheet region of the barrier sheet along fold lines extending in the longitudinal direction onto and bonded to itself.

According to another preferred embodiment, the fold lines extend between front and rear end portions of the barrier sheet, the barrier sheet comprising a main sheet portion and cuff-like portions folded back along the fold lines onto the side facing the wearer's clothes, wherein the tucks are defined by at least parts of the main sheet portion overlapping the cuff-like portions.

According to still further preferred embodiment, the means comprise elastic members attached under tension to the sheet region so as to extend in the longitudinal direction.

According to a yet further preferred embodiment, the barrier sheet elastic members extend between the front and rear end portions and bow in the middle portion to define convex segments towards a longitudinal center line bisecting the barrier sheet in the transverse direction, and the barrier sheet elastic means extend in the longitudinal direction at least in regions defined between the convex segments and the side edges.

### EFFECT OF THE INVENTION

According to the present invention, the barrier sheet is provided in the sheet region thereof defined between the side edges opposed to and spaced from each other in the transverse direction, with the barrier sheet elastic means adapted to prevent the barrier sheet from sagging toward the passage openings. In this way, the barrier sheet should not interfere with smooth movement of body waste into the liquid-absorbent structure.

### BRIEF DESCRIPTION OF THE DRAWINGS

[FIG. 1] Fig. 1 is a perspective view of a first embodiment of the present invention.
[FIG. 2] Fig. 2 is a sectional view taken along the line II-II in Fig. 1.
[FIG. 3] Fig. 3 is a plan view showing the diaper of Fig. 1 provided in a flat opened out arrangement.
[FIG. 4] Fig. 4 is an exploded perspective view of the plan view of the diaper of Fig. 3.
[FIG. 5] Fig. 5 is a partially cutaway scale-enlarged view of the barrier sheet according to a first embodiment.
[FIG. 6] Fig. 6A is a scale-enlarged sectional view taken along the line VI-VI of Fig. 5, showing the barrier sheet laminated on the liquid-absorbent panel and Fig. 6B illustrates the barrier sheet of Fig. 6A wherein the elastic members have been contracted.
[FIG. 7] Fig. 7 is a partially cutaway scale-enlarged view of the barrier sheet according to a second embodiment.
[FIG. 8] Fig. 8 is a scale-enlarged sectional view taken along the line VIII-VIII of Fig. 7, showing the barrier sheet laminated on the liquid-absorbent panel.

### IDENTIFICATION OF REFERENCE NUMERALS USED IN THE DRAWINGS

- 1: diaper
- 2: liquid-absorbent chassis
- 3: front waist region
- 4: rear waist region
- 5: crotch region
- 6: cover sheet
- 7: liquid-absorbent structure
- 8: barrier sheet
- 24: front end portion
- 25: rear end portion
- 26: middle portion
- 27: front passage opening
- 28: rear passage opening
- 34L: barrier sheet elastic member
- 34R: barrier sheet elastic member
- 38: cuff-like portion
- 39: cuff-like portion
- 40: side edges
- 41: side edges
- 42: tuck (anti-sagging means)
- 43: tuck (anti-sagging means)
- 44: lateral portion
- 45: lateral portion
- 46: anti-sagging elastic member (anti-sagging means)
- 47: anti-sagging elastic member (anti-sagging means)
- 51: main sheet portion
- 52: fold line
- 53: fold line

### DESCRIPTION OF THE BEST MODE FOR WORKING OF THE INVENTION

### <Example 1 - First Embodiment>

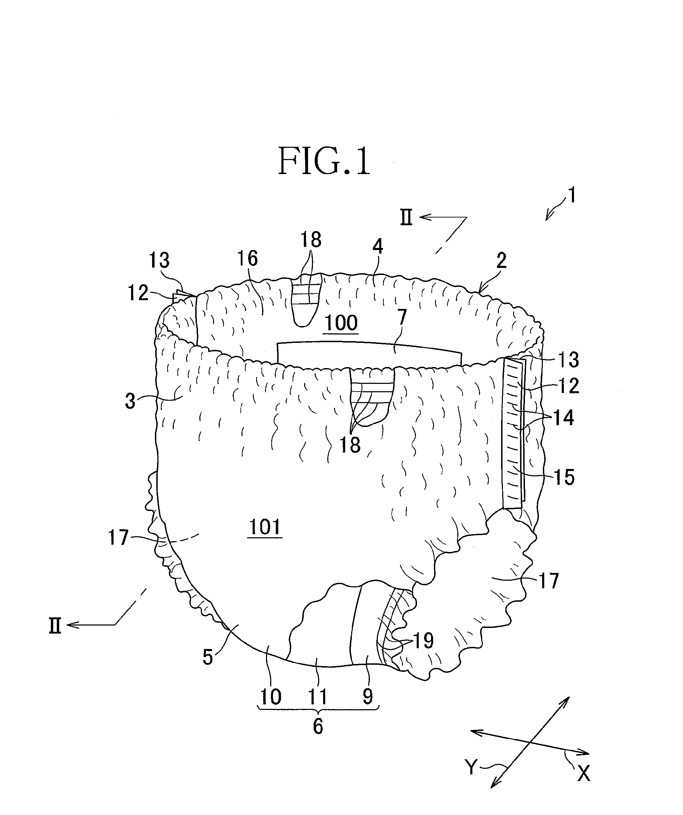

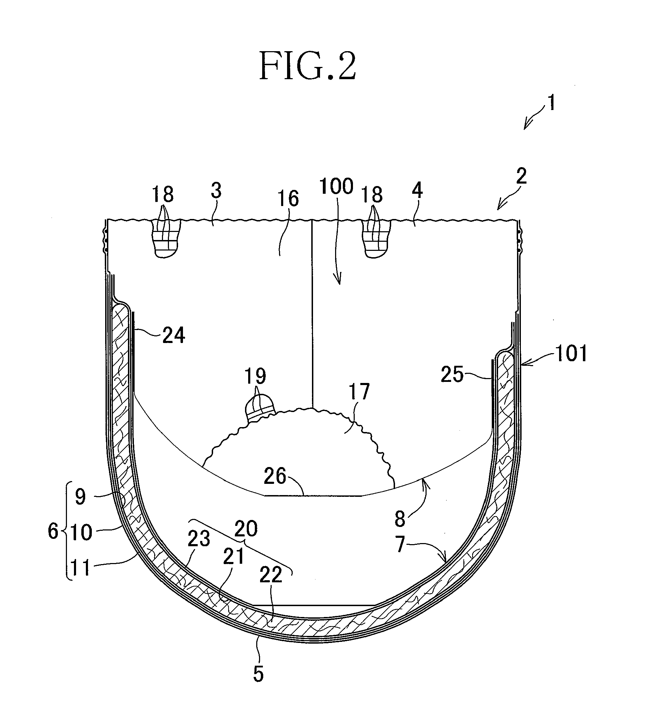

Figs. 1 through to 5 illustrate a first embodiment of the invention. Fig. 1 is a partially cutaway perspective view of a disposable diaper as a typical example of the absorbent article and Fig. 2 is a sectional view taken along the line II-II of Fig. 1. Fig. 1 shows the diaper 1 when worn upon a wearer's body, wherein a transverse direction and a longitudinal direction of the diaper 1 are indicated by arrows X and Y, respectively.

Referring to Fig. 1, the diaper 1 includes a liquid-absorbent chassis 2. The liquid-absorbent chassis 2 has an inner surface 100 facing the wearer's body, an outer surface 101 facing the wearer's garment, a front waist region 3, a rear waist region 4 and a crotch region 5 extending between the front and rear waist regions 3, 4. From a different viewpoint, the diaper 1 comprises a pant-shaped cover sheet 6, a liquid-absorbent structure 7 provided on the inside of the cover sheet 6, i.e., on the inner surface 100 facing the wearer' s body, and a barrier sheet 8 provided on the inside of the liquid-absorbent structure 7. The cover sheet 6 comprises, in turn, an inner sheet 9, an outer sheet 10 and a liquid-impervious sheet 11 sandwiched between these two sheets 9, 10, defining together the front waist region 3, the rear waist region 4 and the crotch region 5. The front and rear waist regions 3, 4 are put flat and joined together along respectively opposed waist side edges 12, 13 at a plurality of joins 14 arranged intermittently along these waist side edges 12, 13 to form seams 15.

The waist side edges 12, 13 are joined together at the joins 14 and thereby a waist-opening 16 is formed in a region surrounded by the front and rear waist regions 3, 4, and at the same time a pair of leg-openings 17 respectively surrounded by the joins 14 and the crotch region 5 are formed. A plurality of waist elastic members 18 extend along a peripheral edge of the waist-opening 16 and a plurality of leg elastic members 19 extend along peripheral edges of the respective leg-openings 17. These elastic members 18, 19 are sandwiched between the inner sheet 9 and the outer sheet 10 and are bonded under tension to at least one of these sheets 9, 10 by means of adhesive (not shown).

Referring to Fig. 2, the liquid-absorbent structure 7 extends in the longitudinal direction Y between the front and rear waist regions 3, 4 but the liquid-absorbent structure 7 effectively functions so far as it is present at least in the crotch region 5. This liquid-absorbent structure 7 includes a liquid-absorbent panel 20 extending on the inner surface of the cover sheet 6.
The liquid-absorbent panel 20 comprises a liquid-absorbent core 22 wrapped with a liquid-absorbent and liquid-spreadable sheet 21, such as tissue paper and an inner sheet 23 adapted to cover at least an area of the upper surface of the core 22 which faces the wearer's skin. The outer surface (bottom surface) of the panel 20 opposed to the inner sheet 23 is covered with the liquid-impervious sheet 11 by the intermediary of the outer sheet 10. The liquid-impervious sheet 11 may sometimes be directly bonded to the bottom surface of the liquid-absorbent panel 20.

In the front and rear waist regions 3, 4, at least a front end portion 24 and a rear end portion 25 of the barrier sheet 8 are bonded to the inner sheet 23 and, if it is necessary, are bonded along lateral portions 44, 45 also. The manner in which the barrier sheet 8 is attached is not limited to the illustrated embodiment and, for example, in the case of the diaper provided with the leak-barrier cuffs, it is also possible to bond the barrier sheet 8 to these leak-barrier cuffs.

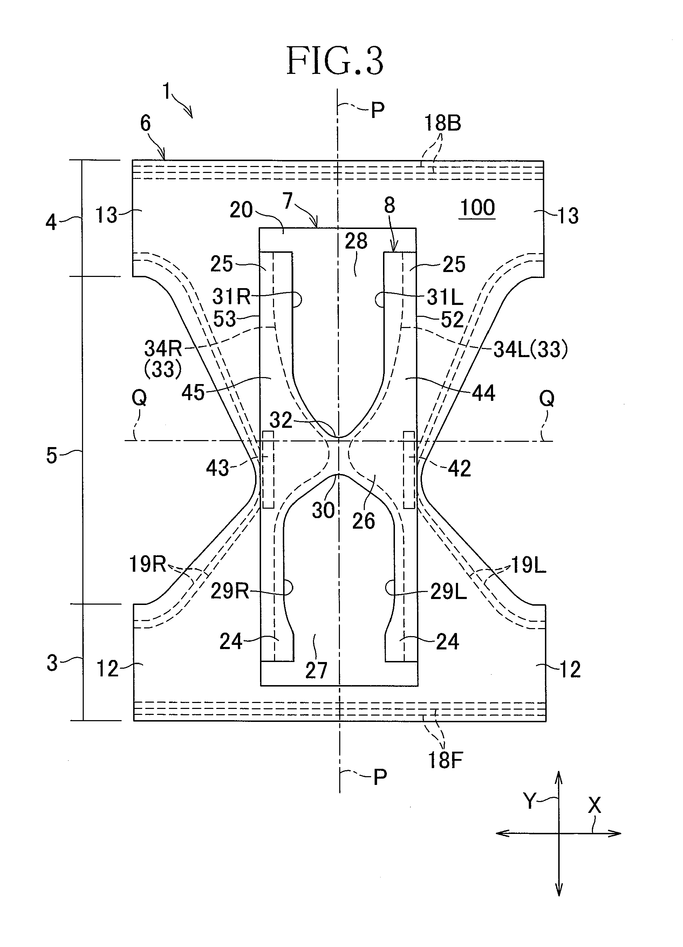

Fig. 3 is a plan view of the diaper 1 obtained by extending out the diaper 1 in the longitudinal direction Y as well as in the transverse direction X after unfastening the front and rear waist regions 3, 4 from each other along each column of joins 14 at the seams 15. In Fig. 3, the elastic members are held in a stretched state so that the diaper 1 may remain in its flattened condition. The expression "left and right" when used in the following description means to be left and right relative to the wearer of the diaper 1. The diaper 1 has a longitudinal center line P-P bisecting a dimension of the diaper 1 in the transverse direction X, and a transverse center line Q-Q bisecting a dimension of the diaper 1 in the longitudinal direction X. The diaper 1 is bilaterally-symmetric about the longitudinal center line P-P.

The cover sheet 6 is formed substantially in a general hourglass-shape and the liquid-absorbent panel 20 is formed in a rectangular shape. The waist elastic members 18 provided on the cover sheet 6 comprise front and rear waist elastic members 18F, 18B as indicated by broken lines while the leg-surrounding elastic members 19 comprise left and right leg-surrounding elastic members 19L, 19R are also indicated by broken lines.

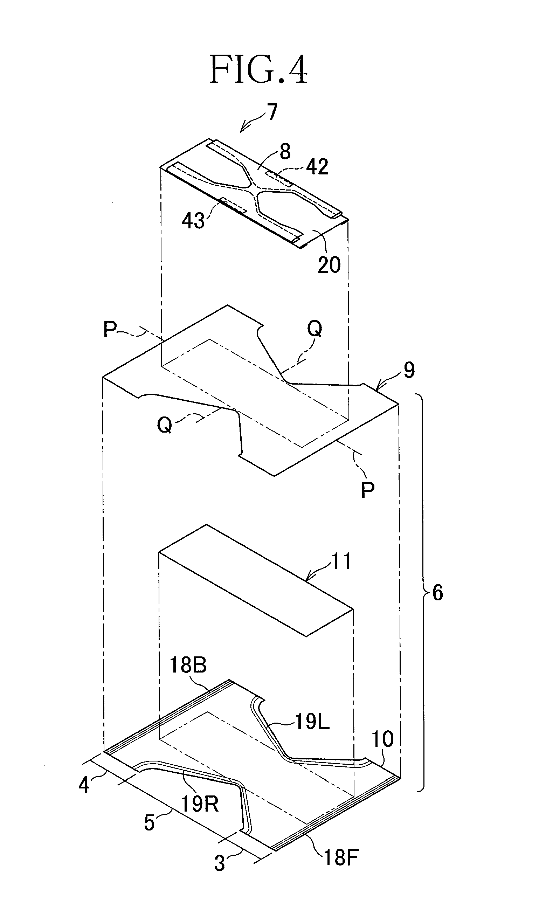

As will be apparent from Fig. 4, which is an exploded perspective view of the diaper 1, the liquid-absorbent panel 20 is laminated on the cover sheet 6. The outer sheet 10 constituting the cover sheet 6 is formed by a fibrous nonwoven fabric or moisture-pervious plastic film of which the inner surface (i.e., upper surface as viewed in Fig. 4) facing the wearer's skin is provided with the waist elastic members 18F, 18B and the leg elastic members 19L, 19R attached under tension thereto by means of hot melt adhesive (not shown). The liquid-impervious sheet 11 formed from the liquid-impervious and moisture-pervious plastic film is attached to the inner surface of the outer sheet 10 by means of adhesion or welding. As has previously been described, this liquid-impervious sheet 11 may be directly bonded to the bottom surface of the liquid-absorbent panel 20 and, in this case, the liquid-impervious sheet 11 preferably is of an area at least sufficient to cover the bottom surface of the liquid-absorbent core 22 from in order to achieve a liquid-impervious effect.

The liquid-impervious sheet 11 is provided on an inner surface with the inner sheet 9 formed from a fibrous nonwoven fabric or moisture-pervious plastic film and having a shape and size the same as those of the outer sheet 10 bonded thereto by means of adhesion or welding. The inner sheet 9 is provided, in turn, on its inner surface with the liquid-absorbent panel 20 bonded thereto. The liquid-absorbent panel 20 is intermittently coated on substantially the entirety of its outer surface (i.e., bottom surface as viewed in Fig. 4) with hot melt adhesive (not shown) by means of which the liquid-absorbent panel 20 is bonded to the inner sheet 9. The liquid-absorbent panel 20 is provided on its inner surface with the barrier sheet 8.

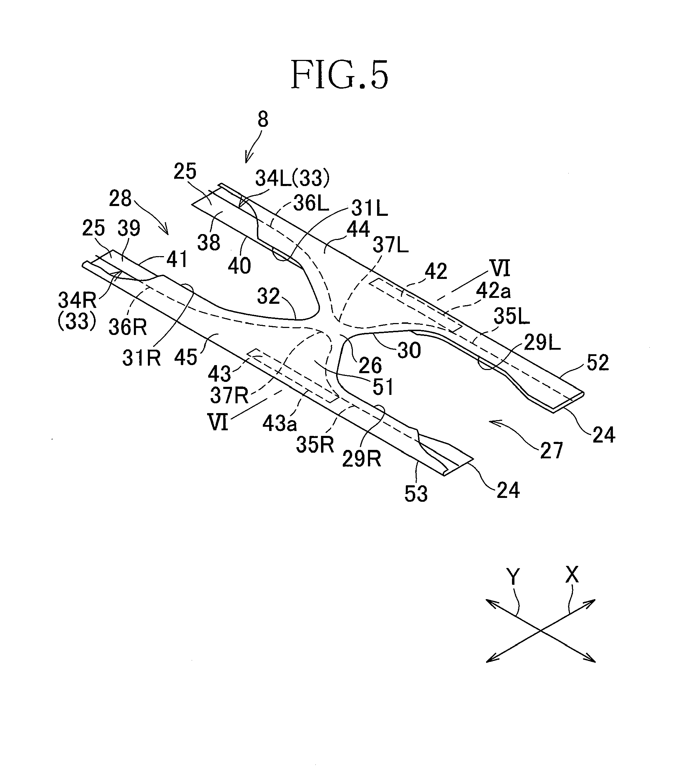

Fig. 5 illustrates the barrier sheet 8 of Figs. 3 and 4 in an enlarged scale. While the remaining members of the diaper 1 are not shown in Fig. 5, it should be assumed that the barrier sheet 8 is affixed to the diaper 1.
The barrier sheet 8 is formed from a sheet material such as fibrous nonwoven fabric, moisture-pervious plastic film or laminate thereof and is preferably liquid-impervious. The barrier sheet 8 comprises lateral portions 44, 45 opposed to and spaced from each other in the transverse direction X, cuff-like portions 38, 39 defined by folding these lateral portions 44, 45 along respective fold lines 52, 53 back onto the side of the liquid-absorbent panel 20 and the remaining portion referred to herein as a main sheet portion 51 facing the wearer's skin. In the barrier sheet 8, front and rear end portions 24, 25 of the respective cuff-like portions 38, 39 are bonded to the inner sheet 23 (See Fig. 2).

The main sheet portion 51 includes a middle portion 26 having the lateral portions 44, 45 opposed to and spaced from each other in the transverse direction X and connecting these lateral portions 44, 45 wherein the middle portion 26 is present in the crotch region 5. Specifically, a substantially U-shaped front passage opening 27 extending from the middle portion 26 toward the front waist region 3 and a substantially U-shaped rear passage opening 28 extending from the middle portion 26 toward the rear waist region 4 are defined. The front passage opening 27 is defined by two inner side edges 29L, 29R of the respective lateral portions 44, 45 and a curved closure edge 30 connecting the inner side edges 29L, 29R with each other, and the rear passage opening 28 is defined by two inner side edges 31L, 31R and a curved closure edge 32 connecting these inner side edges 31L, 31R with each other. The front passage opening 27 and the rear passage opening 28 respectively extend from the front and rear end portions 24, 25 of the barrier sheet 8 toward the middle portion 26 so that the sheet region allocated to come in direct contact with the wearer' s skin may be as limited as possible and undesired stuffiness due to sweat or the other secretions may be prevented. Although not illustrated, it is possible to form the barrier sheet 8 to have substantially the same size and shape as those of the inner sheet 9 defining a part of the cover sheet 6 and/or the front and rear passage openings 27, 28 are closed in the front and rear waist regions 3, 4.

The barrier sheet 8 is provided between the main sheet portion 51 and the cuff-like portions 38, 39 with barrier sheet elastic members 34L, 34R by which the barrier sheet 8 is spaced upward from the liquid-absorbent structure 7 in a thickness direction of the latter. These barrier sheet elastic members 34L, 34R each preferably comprises a single rubber thread extending in the longitudinal direction Y along the lateral portions 44, 45 and immediately along the inner side edges 29L, 29R defining the front passage opening 27 and immediately along inner side edges 31L, 31R defining the rear passage opening 28.

The barrier sheet elastic member 34L comprises first and second segments 35L, 36L extending on opposite end sides as viewed in the longitudinal direction Y and a third segment 37L extending between these first and second segments 35L, 36L. The first and second segments 35L, 36L describe substantially straight paths extending along inner side edges 29L, 31L of the front and rear passage openings 27, 28, respectively, and the third segment 37L describes a path extending along the closure edges 30, 32 in the middle portion 26, so as to be convex toward the longitudinal center line P-P. The barrier sheet elastic member 34R comprises first and second segments 35R, 36R extending on opposite end sides as viewed in the longitudinal direction Y, and a third segment 37R extending between these first and second segments 35R, 36R. The first and second segments 35R, 36R describe substantially straight paths extending along inner side edges 29R, 31R of the front and rear passage openings 27, 28, respectively, and the third segment 37R describes a path extending along the closure edges 30, 32 in the middle portion 26, so as to be convex toward the longitudinal center line P-P. Along the third segment 37L, 37R, a dimension D2 between the barrier sheet elastic members 34L, 34R and the closure edges 30, 32, respectively broaden as the elastic members 34L, 34R come close to the longitudinal center line P-P.

A region of the barrier sheet 8 defined between the barrier sheet elastic member 34L and an inner side edge 40, and between the barrier sheet elastic member 34R and an inner side edge 41, respectively, is formed with tucks 42, 43 functioning to prevent the barrier sheet 8 from sagging and reducing the respective areas of the front and rear passage openings 27, 28. This region of the barrier sheet 8 includes the cuff-like portions 38, 39 assisting this region of the barrier sheet 8 to rise up as the barrier sheet elastic members 34L, 34R contract.
The tucks 42, 43 are defined by the main sheet portion 51 being arranged to be partially flat and then bonded together inclusively of the cuff-like portions 38, 39 by means of heat sealing or hot melt adhesion. The tucks 42, 43 are formed between the third segments 37L, 37R and the side edges 40, 41, respectively. By way of example, the tucks 42, 43 may respectively have a dimension of 4 mm in the transverse direction X and a dimension of 50 mm in the longitudinal direction Y, while a distance from the side edges 40, 41 of the barrier sheet 8 to the outer side edges 42a, 43a of the outer side edges 42a, 43a as measured in the transverse direction X may be set to 7.5 mm.

When the diaper 1 is put on the wearer' s body, the relative position of the diaper 1 to the wearer' s body should be adjusted so that the front passage opening 27 is opposed to the wearer's external genitals, the rear passage opening 28 is opposed to the wearer's anus, and the middle portion 26 comes in contact with the wearer's skin in a region defined between the external genital and the anus. With the diaper 1 put on the wearer's body in this manner, preferably the closure of the edge 30 of the front passage opening 27 is placed aside forward from the transverse center line Q-Q and the closure edge 32 of the rear passage opening 28 is just on or in the vicinity of the transverse center line Q-Q.

With the diaper put on the wearer's body in the manner as has been described above, the liquid-absorbent structure 7 is bowed in the longitudinal direction Y, and simultaneously the above-described sheet region rises up as the barrier sheet elastic members 34L, 34R contract so as to create a space between the barrier sheet 8 and the liquid-absorbent structure 7. In this way, a free region including the middle region of the barrier sheet 8 which is opposed to the inner sheet 23 is spaced from this inner sheet 23 in the thickness direction of the diaper 1 (see Fig. 2). When the barrier sheet 8 rises up, the cuff-like portions 38, 39 are developed in the thickness direction thereof so as to tighten the above-described sheet region of the front and rear end portions 24, 25 of the barrier sheet 8. According to this embodiment, the presence of the cuff-like portions 38, 39 correspondingly increases a height by which the barrier sheet 8 rises up and thereby increases a distance by which the wearer' s skin is spaced from the liquid-absorbent panel 20.

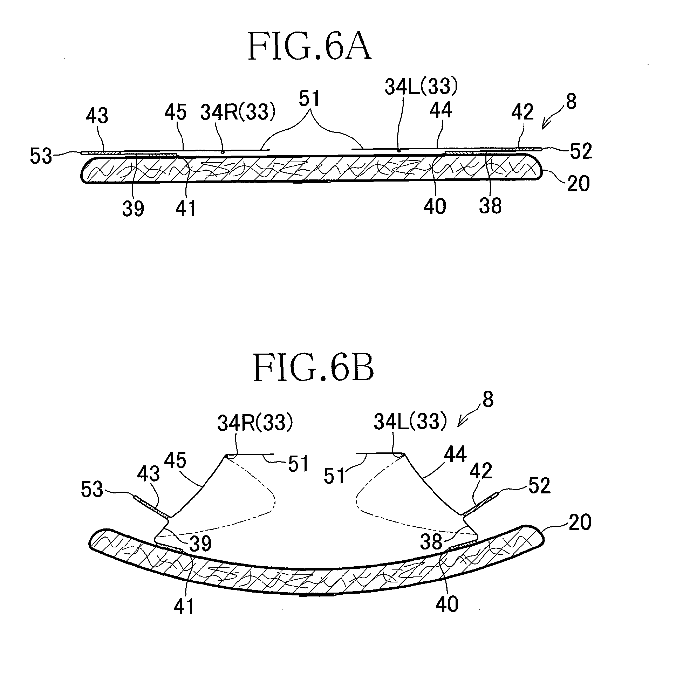

Fig. 6A is a sectional view taken along the line VI-VI of Fig. 5, while Fig. 6B illustrates the barrier sheet having risen up from the state shown in Fig. 6A. For comparison, the sheet region before being provided with the tucks 42, 43 is indicated by imaginary lines. The sheet region defined between the cuff-like portions 38, 39 and the lateral portion 44, 45 rises up toward the wearer's skin as illustrated by Fig. 6B as the barrier sheet elastic members 34L, 34R contract from the state illustrated in Fig. 6A. The tucks 42, 43 provided in this sheet region function to pull and tighten the sheet region in the transverse direction X. The sheet region kept in such a tightened state will prevent the barrier sheet from sagging and reducing the area of the respective passage openings 27, 28. In consequence, the barrier sheet should not interfere with the smooth passage of body waste through the passage openings into the spaces adapted to receive the body waste.
If it is assumed that the barrier sheet 8 is not provided with either the tuck 42 nor the tuck 43, then the sheet region will not be kept in a tightened state, and consequently it will sag so that the areas of the respective passage openings will be reduced and smooth passage of body waste through the respective passage openings into the space adapted to receive body waste will not occur. The liquid-absorbent panel 20 eliminated in Fig. 5 for clarity is illustrated in Fig. 6 so that a positional relationship between the barrier sheet 8 and the liquid-absorbent panel 20 can be seen.

While the tucks 42, 43 are provided in the middle portion 26 according to this embodiment, it is also possible to provide them in any portion other than the middle portion 26. It should be noted, however, that the area of the sheet region in which the barrier sheet elastic members 34L, 34R convexly curve is relatively large and correspondingly apt to sag, possibly reducing the areas of the respective passage openings. In view of such a problem, the tucks are preferably provided in the sheet region having a relatively large area.
While the barrier sheet 8 is provided along the lateral portions 44, 45 with the cuff-like portions 38, 39 in this embodiment, such cuff-like portions 38, 39 are not essential. Instead, the sheet may be nipped from one surface thereof to form fold lines along which the other surface of the sheet may be put flat and bonded together along the respective fold lines so as to form the tucks. In this case, the side edges 43, 44 may be bonded directly to the inner sheet 23.

It is also possible to provide the barrier sheet 8 along the side edges 43, 44 with separate liquid-barrier cuffs serving to prevent body waste from leaking in the transverse direction X. While the cuff-like portions 38, 39 are bonded at the front and rear end portions 24, 25 thereof to the inner sheet 23 according to this embodiment, these cuff-like portions 38, 39 may be bonded to the inner sheet 23 fully along the side edges 40, 41 in the longitudinal direction Y to obtain a pair of liquid-barrier cuffs formed from the cuff-like portions 38, 39. In consequence, it is unnecessary to provide the barrier sheet 8 with any separately prepared liquid-barrier cuffs.

While the barrier sheet 8 is provided with front and rear passage openings 27, 28 according to this embodiment, it may be contemplated that the barrier sheet 8 is provided with only one of these two passage openings. From a viewpoint that the wearer's skin should be protected from contamination with body waste, the barrier sheet 8 is preferably provided with at least the rear passage opening 28.

### <Example 2 - Second Embodiment>

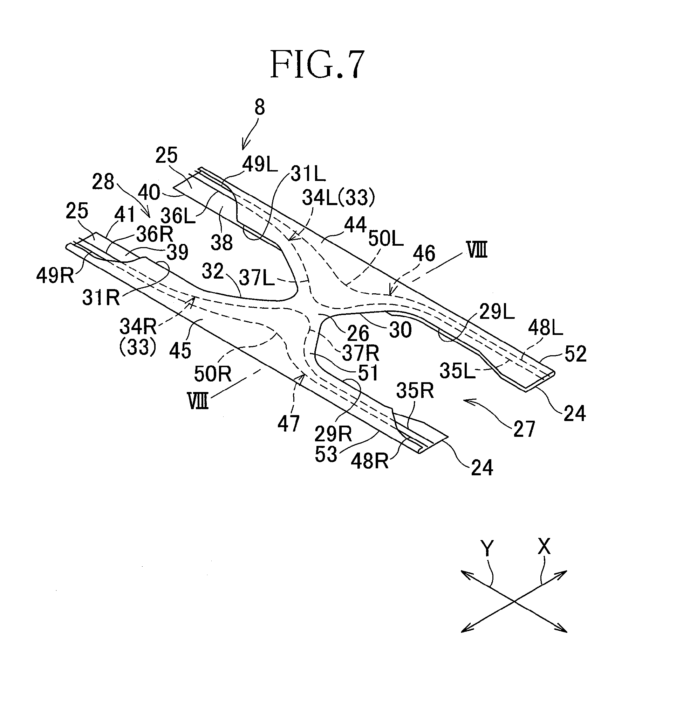

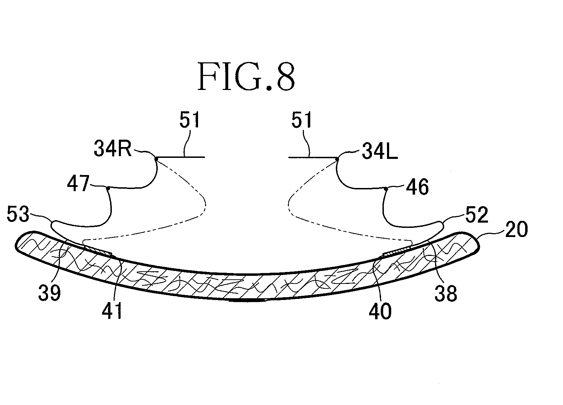

Figs. 7 and 8 illustrate a second embodiment wherein Fig. 7 is a view similar to Fig. 5 showing the first embodiment. This embodiment is **characterized in that** the anti-sagging elastic members 46, 47 are provided as the anti-sagging means. The other constituents are same as those in the first embodiment and as such, details thereof will not be described.

The anti-sagging elastic members 46, 47 are provided between the main sheet portion 51 and the cuff-like portions 38, 39 so as to extend in the longitudinal direction Y and to be permanently bound under tension in this direction Y to the main sheet portion 51 by means of adhesive (not shown). Specifically, these anti-sagging elastic members 46, 47 are provided between the barrier sheet elastic members 34L, 34R and the side edges 40, 41, so as to extend in a substantially parallel direction to the barrier sheet elastic members 34L, 34R. Thus, the anti-sagging elastic members 46, 47 comprise first and second segments 48L, 48R; 49L, 49R extending in the front and rear end portions 24, 25 as viewed in the longitudinal direction Y, and third segments 50L, 50R extending between the first and second segments.

The first and second segments 48L, 48R; 49L, 49R are substantially rectilinear segments extending along the respective side edges 44, 45 and the third segments 50L, 50R extend along the respective curved closure edges 30, 32 convexly toward the longitudinal center line P-P.

Fig. 8 is a sectional view taken along a line VIII-VIII of Fig. 7 and imaginary lines indicate a posture taken by the sheet region on the assumption that none of the anti-sagging elastic members 46, 47 is provided. The liquid-absorbent panel 20 eliminated in Fig. 7 for clarity, is illustrated in Fig. 8 so that a positional relationship between the barrier sheet 8 and the liquid-absorbent panel 20.
The anti-sagging elastic members 46, 47 extend in parallel to the barrier sheet elastic members 34L, 34R and, upon contraction thereof in the longitudinal direction Y, gather the barrier sheet 8 so that the barrier sheet 8 may be tightened in the longitudinal direction Y and thereby the sheet region is pulled outward as viewed in the transverse direction X of Fig. 8. In this way, the anti-sagging elastic members 46, 47 keep the barrier sheet 8 tightened in the longitudinal direction Y and thereby prevent the barrier sheet from sagging and reducing the areas of the respective passage openings.

Arrangement of the anti-sagging elastic members 46, 47 may be appropriately modified or varied, for example, they may be attached to the barrier sheet 8 in rectilinear fashion. It is also possible to replace the anti-sagging elastic members by an elasticized sheet. Furthermore, it is not essential for the anti-sagging elastic members 46, 47 to extend fully to the front and rear end portions 24, 25 of the barrier sheet 8, i.e., these elastic members may effectively function even when these elastic members do not fully extend to the front and rear end portions 24, 25.

## Claims

1. An absorbent article (1) comprising a liquid-absorbent chassis (2) having a longitudinal direction, a transverse direction, a side facing the wearer's body (100), a side facing the wearer's garment (101), a front waist region (3), a rear waist region (4), a crotch region (5) extending between said front and rear waist region (3,4) and a liquid-absorbent structure (7) provided in said crotch region, and a barrier sheet (8) provided on said side facing the article wearer' s body (100) of said liquid-absorbent chassis (2) and adapted to be spaced from said liquid-absorbent chassis (2) wherein said barrier sheet (8) includes barrier sheet elastic members (34L, 34R) attached under tension thereto and extending in said longitudinal direction and a space is formed between said barrier sheet (8) and said liquid-absorbent structure (7) as said liquid-absorbent structure (7) is bowed in said longitudinal direction, said absorbent article being
**characterized in that**:
said barrier sheet (8) comprises a pair of lateral portions (44, 45) opposed to and spaced from each other in said transverse direction, a middle portion (26) connecting said lateral portions (44, 45) to each other, front and rear passage openings (27, 28) defined by said lateral portions (44, 45) and said middle portion (26) through which body waste can be guided into said space and said lateral portions (44, 45) are attached at front and rear end portions (24, 25) thereof to said front and rear waist regions (3, 4);
said barrier sheet elastic members (34L, 34R) are attached to said lateral portions (44, 45) and extend along said passage openings; and
said barrier sheet (8) is provided with means adapted to prevent a sheet region of said barrier sheet extending outside said barrier sheet elastic members (34L, 34R) as viewed in said transverse direction from sagging toward said passage openings.

2. The absorbent article (1) as claimed in Claim 1, wherein said means adapted to prevent a sheet region of said barrier sheet extending outside said barrier sheet elastic members comprise tucks (42, 43) formed by at least partially folding back said sheet region of said barrier sheet (8) along fold lines extending in said longitudinal direction onto and bonded to itself.

3. The absorbent article (1) as claimed in Claim 2, wherein:
said fold lines (52, 53) extend between front and rear end portions of said barrier sheet (8);
said barrier sheet (8) comprises a main sheet portion and cuff-like portions folded back along said fold lines onto said side facing the wearer's garment (101); and
said tucks (42, 43) are defined by at least part of said main sheet portion overlapping said cuff-like portions.

4. The absorbent article (1) as claimed in Claim 1, wherein said means adapted to prevent a sheet region of said barrier sheet extending outside said barrier sheet elastic member comprise elastic members attached under tension to said sheet region so as to extend in said longitudinal direction.

5. The absorbent article (1) as claimed in any one of Claims 1 to Claim 4, wherein:
said barrier sheet elastic members (34L, 34R) extend between said front and rear end portions and bow in the middle portion to define convex segments toward a longitudinal center line bisecting said barrier sheet (8) in a transverse direction; and wherein said means adapted to prevent a sheet region of said barrier sheet extending outside said barrier sheet elastic member extends in the longitudinal direction at least in regions defined between said convex segments and said side edges.
